# EUROPEAN PATENT SPECIFICATION

(11) **EP 2 971 106 B1**
(45) Date of publication and mention of the grant of the patent: **26.02.2020**
(21) Application number: 14723520.4
(22) Date of filing: 10.03.2014
(51) Int. Cl.: C12Q 1/6844

(54) **METHODS FOR TRUE ISOTHERMAL STRAND DISPLACEMENT AMPLIFICATION**
VERFAHREN FÜR ECHTE ISOTHERMALE STRANGVERSCHIEBUNGSAMPLIFIKATION
PROCÉDÉS D'AMPLIFICATION ISOTHERME PAR DÉPLACEMENT DE BRIN EN TEMPS RÉEL

(30) Priority: 11.03.2013 US 201361776256 P
(43) Date of publication of application: 20.01.2016
(73) Proprietor: ELITechGroup, Inc., Logan, UT 84321 (US)
(72) Inventor: BELOUSOV, Yevgeniy, S., Mill Creek, WA 98012 (US); ALABEYEV, Boris, Lynnwood, WA 98087 (US); SCARR, Noah, Seattle, WA 98133 (US)
(74) Representative: Walker, Ross Thomson
(86) International application number: PCT/US2014/022534
(87) International publication number: WO 2014/164479

(56) References cited:
- EP-A1- 0 678 582
- WO-A2-2008/002920
- US-A- 6 063 604
- US-A1- 2010 255 546
- ANGELIKA NIEMZ ET AL: "Point-of-care nucleic acid testing for infectious diseases", TRENDS IN BIOTECHNOLOGY, vol. 29, no. 5, 4 March 2011 (2011-03-04), pages 240-250, XP028194817, ISSN: 0167-7799, DOI: 10.1016/J.TIBTECH.2011.01.007 [retrieved on 2011-01-25] cited in the application
- EHSES S ET AL: "Optimization and design of oligonucleotide setup for strand displacement amplification", JOURNAL OF BIOCHEMICAL AND BIOPHYSICAL METHODS, AMSTERDAM, NL, vol. 63, no. 3, 30 June 2005 (2005-06-30), pages 170-186, XP027670200, ISSN: 0165-022X [retrieved on 2005-06-30] cited in the application

## Description

### BACKGROUND

This application claims priority to U.S. Provisional Patent Application Serial No. 61/776,256, filed March 1 1, 2013, entitled "Methods for True Isothermal Strand Displacement Amplification,".

This disclosure pertains to methods for isothermal strand displacement amplification that accomplishes efficient primer extension amplification with target specific primers and does not require pre-denaturation.

Isothermal amplification requires single stranded targets for efficient primer extension. Helicase dependent amplification of nucleic acids also requires helicase enzyme for unwinding double strands to allow amplification with a DNA polymerase (US Patent No. 7282328). Exponential strand displacement amplification ("SDA") as described in US Patent No. 5,455,166 requires an initial denaturation of the target into single-stranded DNA (ssDNA), generation of hemiphosphorothioate sites which allow single strand nicking by restriction enzymes, and extension by a polymerase lacking 5'-3' exonuclease activity. Raising the temperature of the reaction to approximately 95°C to render double strands into single strands is required to permit binding of the primers to the target strands. State of the art SDA amplification requires the denaturation of the target at elevated temperature to yield ssDNA for strand displacement isothermal amplification (US2010/255546 A1; US6063604 A; EP0678582 A1; WO2008/002920 A2).

The use of a nicking enzyme to cleave one of the strands of a target instead of the generation of hemiphosphorothioate sites in SDA amplification was described in (Ehses et al, J. Biochem. Biophys. Methods. 63: 170-86 (2005)). The design of primers to reduce non-predictable byproducts was also described. Denaturation at 95°C was required by Ehses et al. after the addition of target and before the addition of any enzymes. Nicking enzyme SDA amplification without denaturation of target at 95°C was reported in U.S. Patent Application Publication No. 2009/0092967. However, a limitation of the latter method is that a limited number of nicking enzymes are available and quite often no natural nicking site is present in a target region of interest. An abasic site endonuclease amplification assay was disclosed in U.S. Patent Application Publication No. 2004/0101893. The use of this assay as a post amplification detection system in combination with other amplification systems was also disclosed. These assays require a denaturation step of dsDNA.

It is known in the art that double stranded (ds) nucleic acid can be denatured in different ways. Heat denaturation is state of the art to separate ds DNA into single strands. Native DNA denatures at about 85°C. (White, Handler and Smith, Principles of Biochemistry 5th Edition, McGraw-Hill Kogakush, Ltd, pages 192-197, 1993). Early on, it was established that primer extension in amplification required the binding of a primer to a single strand DNA strand. This was preferably achieved by heating the sample at about 95°C (M Panaccio and A Lew. PCR based diagnosis in the presence of 8% (v/v) blood. Nucleic Acids Res., 19: 1151 (1991)). It was recently reported that Watson-Crick pairs in naked DNA spontaneously flip into Hoogstein pairs under ordinary conditions, suggesting that DNA breathes (Fran-Kamentskii. Artificial DNA; PNA & XNA, 2:1, 1-3 (2011)).

A few nucleases cut just one strand of DNA thereby introducing a nick into DNA (Besnier and Kong, EMBO Reports, 21: 782-786 (2001)). Most such proteins are involved in DNA repair and other DNA-related metabolism and cannot easily be used to manipulate DNA. They usually recognize long sequences and associate with other proteins to form active complexes that are difficult to manufacture (Higashitani et al., J. Mol. Biol., 237: 388-4000 (1994)). Single strand nicking endonucleases which nick only one strand of the DNA double strands and traditional restriction endonucleases are listed and updated in the REBASE Database (rebase.neb.com; Roberts et al., Nucl. Acids Res., 31: 418-420 (2003)). Engineering of a nicking endonuclease has been described (Xu et al, PNAS 98: 12990-12995 (2001)).

Other methods using isothermal amplification have been disclosed recently (Niemz et al., Trends in Biotechnol., 29:240-250 (2011)). However, these amplification methods also utilize thermal or other denaturation.

In a first aspect of the present invention there is provided an isothermal strand displacement amplification method, the method comprising:
(a) contacting a double stranded genomic nucleic acid having a target nucleic acid sequence with an amplification reaction mixture comprising:
   a forward primer and a reverse primer,
      wherein the forward primer has the formula:

         A-B,

         wherein B comprises a portion of the forward primer that is complementary to the target nucleic acid sequence, and wherein A comprises a portion of the forward primer that is non-complementary to the target nucleic acid sequence and comprises a forward nicking enzyme recognition sequence,
      wherein the reverse primer has the formula:

         A'-B',

         wherein B' comprises a portion of the reverse primer that is complementary to the target nucleic acid sequence, and wherein A' comprises a portion of the reverse primer that is non-complementary to the target nucleic acid sequence and comprises a reverse nicking enzyme recognition sequence, and
      wherein the forward primer and the reverse primer comprise sequences optimized by software for specific hybridization and efficient elongation,
   a polymerase enzyme having strand displacement activity, and
   a nicking enzyme specific for the nicking enzyme recognition sequence;
(b) incubating the amplification reaction mixture and the double stranded genomic nucleic acid under amplification conditions suitable for amplification of the target nucleic acid to produce an amplified target nucleic acid,
   wherein isothermal amplification of the target nucleic acid occurs at a temperature between about 45°C and about 55°C and the double stranded genomic nucleic acid is not denatured prior to amplification; and
(c) detecting the amplified target nucleic acid.

### SUMMARY

The present invention relates generally to an isothermal assay which utilizes the advantages of target nucleic acid amplification without the requirement for dsDNA denaturation. The present methods enable efficient detection of target nucleic acids with exquisite specific amplification. The present disclosure unexpectedly determined that primers designed according to a particular method allow efficient primer extension amplification of target specific primers without pre-denaturation. Generally, the present disclosure provides methods, primers and probes for the isothermal amplification without denaturation of nucleic acid targets for polymerase primer extension (isothermal strand displacement amplification ("iSDA")) in samples including biological samples (e.g., blood, nasopharyngeal or throat, swab, wound swab, or other tissues). The nucleic acid targets may be double stranded or they may be single stranded, such as RSV virus. RNA targets may be single stranded or double stranded.

The method described herein utilizes primer oligonucleotides that allow primer extension without denaturation of nucleic acid targets. In some examples the primers have modified bases to improve stability or to eliminate primer self-association. In one embodiment modified bases are used to limit primer self-association.

In certain examples the primer comprises a 5'-non-complementary tail wherein said tail further comprises a nicking enzyme specific sequence.

In the methods described herein, the nucleic acids present in a clinical or test sample obtained from a biological sample or tissue suspected of containing a clinical target (microorganisms or tissue, for example) are extracted with methods known in the art. The target nucleic acids are amplified without denaturation and detected. More specifically the target specific primers contain a sequence specific for target and a non-target complementary 5'-tail, wherein the tail contains a sequence specific for a nicking enzyme when hybridized to its complementary sequence. At least one amplification cycle provides a double stranded amplicon containing a nicking site which allows strand displacement in a second amplification cycle. The amplified nucleic acid can be detected by a variety of state of the art methods including fluorescence resonance energy ("FRET"), radiolabels, lateral flow, enzyme labels, and the like.

The methods described herein also include methods for the design of primers allowing amplification of at least one cycle of amplification without denaturation of duplex DNA target.

In certain methods provided herein the methods comprise the detection of iSDA or RT-iSDA amplified targets by lateral flow.

Those skilled in the art will appreciate that the present disclosed amplification method can be performed in combination with other methods. In one embodiment the amplification method described in U.S. Patent Application Publication No. 2009/0092967 can be combined with the method of the present disclosure.

This disclosure provides an isothermal method for specifically detecting a nucleic acid sequence in a biological sample from an individual. The disclosure also provides oligonucleotide primers and probes comprising nucleotide sequences characteristic of specific genomic nucleic acid sequences. The method includes performing isothermal amplification without a denaturation step prior to amplification. The amplification step includes contacting the sample nucleic acid with pairs of primers to produce amplification product(s) if the specific genomic nucleic acid target is present. The preferred primers target a specific region of a specific target gene. Each of the preferred primers has a 5'-oligonucleotide tail non-complementary to the target where said non-complementary tail contains a sequence when hybridized to a complementary sequence contains a nicking enzyme cleavage site. The oligonucleotide probes detect the amplified target directly or indirectly. The preferred oligonucleotide probe is a 5'-minor groove binder-fluorophore-oligonucleotide-quencher-3' conjugate that fluoresces on hybridization to its complementary amplified target. In some embodiments one or more primer is labeled. In some embodiments a double strand binding fluorescent dye is used. In some embodiments one or more bumper oligonucleotides are provided. In some embodiments the probe(s) is omitted. In some embodiments the amplified target is captured on a solid support or membrane and detected by a labeled probe. In some embodiments the primer concentrations are present in different concentrations. In some embodiments an internal control is provided.

### BRIEF DESCRIPTION OF THE DRAWINGS

Figure 1 shows a schematic of an example of dual capture and detection of iSDA amplified amplicon by pDNA immobilized on a solid surface;
Figure 2 shows an example of real-time iSDA amplification of different concentrations of the *ldh1* gene with fluorescence detection utilizing a Pleiades probe;
Figure 3 shows an example of lateral flow colorimetric detection of an *ldh1* iSDA amplified amplicon with the approach provided in Figure 1;
Figure 4 shows an example of real-time iSDA amplification of two different *mecA* designed assays with fluorescence detection utilizing a Pleiades probe;
Figure 5 shows an example of real-time iSDA amplification with different polymerases;
Figure 6 shows an example of gel analysis of the valuation of Nt.Alw I on PCR Amplified target containing Nt.Alw I cleavage site;
Figure 7 shows an example of lateral flow detection of iSDA biplex-amplified *ldh1* and IC amplicons;
Figure 8 shows a schematic representation of a primer containing a complementary- and non-complementary-sequence;
Figure 9 shows the probe specific iSDA detection and differentiation of *ldh1* gene in *S. aureus* and of *S. epidermis*;
Figure 10 shows the specific real-time iSDA amplification of *S. aureus* nucleic acid extracted with five different extraction methods;
Figure 11 shows the results of amplification reactions comparing amplification with primers and probes optimized for use in the present isothermal strand displacement amplification method and traditional primers and probes;
Figure 12 shows the specific reverse transcriptase-iSDA (RT-iSDA) amplification of Respiratory syncytial virus (RSV) extracted RNA nucleic acid using both real-time fluorescence detection and post-amplification lateral flow detection; and
Figure 13 shows the real-time iSDA amplification of native and denatured *Plasmodium falciparum* DNA.

### DETAILED DESCRIPTION OF PREFERRED EMBODIMENTS

### I. General

Generally, the present disclosure provides methods, primers and probes for the isothermal amplification and detection, without denaturation, of double stranded nucleic acid targets for polymerase strand displacement amplification ("iSDA"). The methods and compositions disclosed are highly specific for nucleic acid targets with high sensitivity, specificity and speed that allow detection of clinical relevant target levels. The methods and compositions can easily be used to amplify or detect nucleic acid targets in biological samples.

According to Ehses et al. (J. Biochem. Biophys. Methods. 63:170-86 (2005),), primers can be designed using the Vienna Folding Package (tbi.univie.ac.at./ivo/RNA/) that identifies analyzes sequences that allowing one to minimize the accumulation of non-predictable byproducts especially for longer incubation times and low concentrations of initial template DNA. More specifically, the Vienna Folding Package is a software product that predicts a secondary structure of the primers based on the calculations of the minimum free energy of the hybridization reaction and calculates the probabilities of alternative DNA/DNA duplex structures. Primers designed using software such as the Vienna Folding Package are considered to have an improved hybridization stringency, and thus permit efficient elongation of a target sequence. The Tₘ of the selected primers can then be adjusted by calculation with a preferred software package, such as the Eclipse Design Software 2.3 (Afonina et al., Single Nucleotide Polymorphism Detection with fluorescent MGB Eclipse Systems in A-Z of Quantitative PCR, Ed. S. A. Bustin, International University Line, La Jolla, CA, pages 718-731 and XII-XIII, 2004; see also U.S. Patent Nos. 6683173 and 7751982). The software adjusts the Tm of the primers for optimum extension as well, by calculating duplex stabilities using an algorithm applying a nearest-neighbor model for duplex formation thermodynamics for each of the neighboring base pairs. Each nearest neighbor thermodynamic parameter defines a thermodynamic contribution of two corresponding neighboring bases. A preferred oligonucleotide primer sequence is then selected having the desired duplex stability. The primers can also be designed, if necessary or desired, to include modified bases (see US 7,045,610; US 6,127,121; US 6,660,845; US 5,912,340 and US Application Publication No. 2010/057862. In the case of probes or MGB probes, the same software package (such as Eclipse Design Software 2.3) can be used.

### II. Definitions

A "sample" as used herein refers to a sample of any source which is suspected of containing a target sequence. These samples can be tested by the methods described herein. A sample can be from a laboratory source or from a non-laboratory source. A sample may be suspended or dissolved in liquid materials such as buffers, extractants, solvents, and the like. Samples also include biological samples such as plant, animal and human tissue or fluids such as whole blood, blood fractions, serum, plasma, cerebrospinal fluid, lymph fluids, milk, urine, various external secretions of the respiratory, intestinal, and genitourinary tracts, tears, and saliva; and biological fluids such as cell extracts, cell culture supernatants, fixed tissue specimens, and fixed cell specimens. Samples include nasopharyngeal or throat swabs, stools, wound or rectal swabs. Biological samples may also include sections of tissues such as biopsy and autopsy samples or frozen sections taken for histological purposes. A biological sample is obtained from any animal including, e.g., a human. A biological sample may include human and animal pathogens that includes microbes or microorganisms such as a viruses, bacteria, or fungi that causes disease in humans. Biological samples may further also include products of gene mutated-metabolic disorders.

The terms "flap primer" or "overhang primer" refer to a primer comprising a 5' sequence segment non-complementary to a target nucleic acid sequence, wherein said tail further comprises a nicking enzyme specific sequence and a 3' sequence segment complementary to the target nucleic acid sequence The flap primers are suitable for primer extension or amplification of the target nucleic acid sequence The primers may comprise one or more non-complementary or modified nucleotides (e.g., pyrazolopyrimidines as described in US 7,045,610) at any position including, e.g., the 5' end.

The term "isothermal strand displacement amplification" ("iSDA") refers to primer extension using a primer that comprises a 5' sequence segment non-complementary to a target nucleic acid sequence, wherein said tail may further comprise a nicking enzyme specific sequence and a 3' sequence segment complementary to the target nucleic acid sequence.

The term "fluorescent generation probe" refers either to a) an oligonucleotide having an attached minor groove binder, fluorophore, and quencher, b) an oligonucleotide having an attached fluorophore, and quencher, c) an oligonucleotide having an attached minor groove binder, and fluorophore, d) an oligonucleotide having an attached fluorophore and quencher, e) an oligonucleotide having an attached fluorophore, or f) a DNA binding reagent. The probes may comprise one or more non-complementary or modified nucleotides (e.g., pyrazolopyrimidines as described in US 7,045,610) at any position including, e.g., the 5' end. In some embodiments, the fluorophore is attached to the modified nucleotide. In some embodiments the probe is cleaved to yield a fluorescent signal.

Preferably, modified bases increase thermal stability of the probe-target duplex in comparison with probes comprised of only natural bases (i.e., increase the hybridization melting temperature of the probe duplexed with a target sequence). Modified bases can decrease probe and primer self-association compared to only normal bases. Modified bases include naturally-occurring and synthetic modifications and analogues of the major bases such as, for example, hypoxanthine, 2-aminoadenine, 2-thiouracil, 2-thiothymine, inosine, 5-N⁴-ethenocytosine, 4-aminopyrrazolo[3,4-d]pyrimidine and 6-amino-4-hydroxy-[3,4-d]pyrimidine. Any modified nucleotide or nucleotide analogue compatible with hybridization of probe with a nucleic acid conjugate to a target sequence is useful, even if the modified nucleotide or nucleotide analogue itself does not participate in base-pairing, or has altered base-pairing properties compared to naturally-occurring nucleotides. Examples of modified bases are disclosed in U.S. Pat. Nos. 7,045,610; 5,824,796; 6,127,121; 5,912,340; and PCT Publications WO 01/38584; WO 01/64958. Preferred modified bases include 5-hydroxybutynyl uridine for uridine; 4-(4,6-Diamino-¹H-pyrazolo[3,4-d]pyrimidin-3-yl)-but-3-yn-1-ol, 4-amino-¹H-pyrazolo[3,4-d]pyrimidine, and 4-amino-¹H-pyrazolo[3,4-d]pyrimidine for adenine; 5-(4-Hydroxy-but-1-ynyl)-1H-pyrimidine-2,4-dione for thymine; and 6-amino-¹H-pyrazolo[3,4-d]pyrimidin-4(5H)-one for guanine. Particularly preferred modified bases are "Super A®: 4-(4,6-Diamino-1H-pyrazolo[3,4-d]pyrimidin-3-yl)-but-3-yn-1-ol," "Super G®: 4-hydroxy-6-amino pyrazolopyrimidine" (www.elitechgroup.com) and "Super T®: 5-(4-hydroxy-but-1-ynyl)-1H-pyrimidine-2,4-dione". "Super-D™: 3-Alkynyl pyrazolopyrimidine" analogues as universal bases are disclosed in U.S. Patent Application Publication No. 2012/0244535.

The terms "fluorescent label" or "fluorophore" refer to compounds with a fluorescent emission maximum between about 400 and about 900 nm. These compounds include, with their emission maxima in nm in brackets, Cy2™ (506), GFP (Red Shifted) (507), YO-PRO™-1 (509), YOYO™-1 (509), Calcein (517), FITC (518), FluorX™ (519), Alexa™ (520), Rhodamine 110 (520), 5-FAM (522), Oregon Green™ 500 (522), Oregon Green™ 488 (524), RiboGreen™ (525), Rhodamine Green™ (527), Rhodamine 123 (529), Magnesium Green™ (531), Calcium Green™ (533), TO-PRO™-1 (533), TOTO®-1 (533), JOE (548), BODIPY® 530/550 (550), Dil (565), BODIPY® 558/568 (568), BODIPY® 564/570 (570), Cy3™ (570), Alexa™ 546 (570), TRITC (572), Magnesium Orange™ (575), Phycoerythrin R&B (575), Rhodamine Phalloidin (575), Calcium Orange™ (576), Pyronin Y (580), Rhodamine B (580), TAMRA (582), Rhodamine Red™ (590), Cy3.5™ (596), ROX (608), Calcium Crimson™ (615), Alexa™ 594 (615), Texas Red® (615), Nile Red (628), YO-PRO™-3 (631), YOYO™-3 (631), R-phycocyanin (642), C-Phycocyanin (648), TO-PRO™-3 (660), TOTO®-3 (660), DiD DilC(5) (665), Cy5™ (670), Thiadicarbocyanine (671), and Cy5.5 (694). Additional fluorophores are disclosed in PCT Patent Publication No. WO 03/023357 and U.S. Patent No. 7,671,218. Examples of these and other suitable dye classes can be found in Haugland et al., Handbook of Fluorescent Probes and Research Chemicals, Sixth Ed., Molecular Probes, Eugene, Ore. (1996); U.S. Patent Nos. 3,194,805; 3,128,179; 5,187,288; 5,188,934; 5,227,487, 5,248,782; 5,304,645; 5,433,896; 5,442,045; 5,556,959; 5,583,236; 5,808,044; 5,852,191; 5,986,086; 6,020,481; 6,162,931; 6,180,295; and 6,221,604; EP Patent No. 1408366; Smith et al., J. Chem. Soc. Perkin Trans. 2:1195-1204 (1993); Whitaker, et al., Anal. Biochem. 207:267-279 (1992); Krasoviskii and Bolotin, Organic Luminescent Materials, VCH Publishers, NY. (1988); Zolliger, Color Chemistry, 2nd Edition, VCH Publishers, NY. (1991); Hirschberg et al., Biochemistry 37:10381-10385 (1998); Fieser and Fieser, REAGENTS FOR ORGANIC SYNTHESIS, Volumes 1 to 17, Wiley, US (1995); and Geiger et al., Nature 359:859-861 (1992). Still other dyes are provided via online sites such as www.zeiss.com. Phosphonate dyes are disclosed in co-owned US 7,671,218 and US 7,767,834.

There is extensive guidance in the art for selecting quencher and fluorophore pairs and their attachment to oligonucleotides (Haugland, 1996; U.S. Patent Nos. 3,996,345 and 4,351,760 and the like). Preferred quenchers are described in U.S. Patent No. 6,727,356. Other quenchers include bis azo quenchers (U.S. Patent No. 6,790,945) and dyes from Biosearch Technologies, Inc. (provided as Black Hole™ Quenchers: BH-1, BH-2 and BH-3 quenchers), Dabcyl, TAMRA and carboxytetramethyl rhodamine.

The term "linker" refers to a moiety that is used to assemble various portions of the molecule or to covalently attach the molecule (or portions thereof) to a solid support, surface or membrane. Typically, a linker or linking group has functional groups that are used to interact with and form covalent bonds with functional groups in the ligands or components (e.g., fluorophores, oligonucleotides, minor groove binders, or quenchers) of the conjugates described and used herein. Examples of functional groups on the linking groups (prior to interaction with other components) include -NH2, -NHNH₂,-ONH₂, -NHC=(O)NHNH₂, -OH, and -SH. The linking groups are also those portions of the molecule that connect other groups (e.g., phosphoramidite moieties and the like) to the conjugate. Additionally, a linker can include linear or acyclic portions, cyclic portions, aromatic rings, and combinations thereof.

The term "solid support" refers to any support that is compatible with oligonucleotide attachment, including, for example, glass, controlled pore glass, polymeric materials, polystyrene, beads, coated glass, and the like.

Lateral flow assay technology is well known in the art and is performed on strips of porous paper or sintered polymer see for example US 6,485,982, US 7,799,554, and US 7,901,623.

In the description herein, the abbreviations MGB, FL, Q, CPG, and ODN refer to "minor groove binder," "fluorescent label" or "fluorophore," "quencher," "controlled pore glass" (as an example of a solid support), and "oligonucleotide" moieties or molecules, respectively, and in a manner which is apparent from context. The terms "probe" and "conjugate" are used interchangeably and refer to an oligonucleotide having an attached minor groove binder, fluorophore, and quencher.

The terms "oligonucleotide," "nucleic acid," and "polynucleotide" are used interchangeably herein. These terms refer to a compound comprising nucleic acid, nucleotide, or its polymer in either single- or double-stranded form, e.g., DNA, RNA, analogs of natural nucleotides, and hybrids thereof. The terms encompass polymers containing modified or non-naturally-occurring nucleotides, or to any other type of polymer capable of stable base-pairing to DNA or RNA including, but not limited to, peptide nucleic acids as described in Nielsen et al., Science, 254:1497-1500 (1991), bicyclo DNA oligomers as described in Bolli et al., Nucleic Acids Res., 24:4660-4667 (1996), and related structures. Unless otherwise limited, the terms encompass known analogs of natural nucleotides that hybridize to nucleic acids in a manner similar to naturally-occurring nucleotides. Examples of such analogs include, without limitation, phosphorothioates, phosphoramidates, methyl phosphonates, chiral-methyl phosphonates, 2-O-methyl ribonucleotides, and peptide-nucleic acids (PNAs). A "subsequence" or "segment" refers to a sequence of nucleotides that comprise a part of a longer sequence of nucleotides. In some embodiments, nucleotides may include analogs of natural nucleotides which exhibit preferential binding to nucleotides other than naturally occuring DNA or RNA; an example of such nucleotides is pDNA (Eschenmoser et al, Helvetica Chimica Acta, "Why Pentose- and Hexose-Nucleic Acids?", pp. 76: 2161-2183 (1993)).

The term "Nicking Enzyme (or nicking endonuclease)" describes an enzyme that cuts one strand of a double-stranded DNA at a specifically recognition recognized nucleotide sequences known as a nicking site. Such enzymes hydrolyse (cut) only one strand of the DNA duplex, to produce DNA molecules that are "nicked", rather than cleaved. These nicking enzymes include N.Alw I, Nb.BbvCl, Nt.BbvCl, Nb.BsmI, Nt.BsmAI, Nt.BspQI, Nb.BsrDI, Nt.BstNBI, Nb.BstsCI, Nt.CviPII, Nb.Bpu10I, Nt.Bpu10I and Nt.Bst9I which are commercially available from www.neb.com, www.fermentas.com and www.sibenzyme.com, respectively. The New England Biolabs REBASE website (rebase.neb.com/cgi-bin/azlist?nick) lists 917 nicking enzymes. Designing of artificial nicking endonucleases on the basis of restriction endonucleases was reviewed by Zheleznaya et al., Biochemistry (Mosc). 74:1457-66 (2009). "Nicking Enzyme" also includes engineered enzymes that cut one strand of a double stranded DNA, for example, zinc finger nucleases.

The term "Lateral Flow" describes a porous membrane capable of nonabsorbent lateral flow used as assay substrate; a member of the binding pair is affixed in an indicator zone defined in the substrate. The sample is applied at a position distant from the indicator zone and permitted to flow laterally through the zone; any analyte in the sample is complexed by the immobilized specific binding member, and detected. Lateral flow utilizing immuno-binding pairs is well known in the art (US 4,943,522). Lateral flow using DNA binding pairs was disclosed in US US7488578. pDNA binding pairs are disclosed in co-owned US application 2012-0015358 A1. Biotin-streptavidin affinity pairs are well known in the art and commercially available. Streptavidin-coated label may be a covalent or adsorptively bound streptavidin or other biotin-binding species, and the label may be a polystyrene nanoparticle doped with fluorescent or visible dye, a carbon black nanoparticle, a metal colloid, or other species detectable by fluorescence, radioactivity, magnetism, or visual acumen. The lateral flow buffer may be an aqueous suspension containing detergents, proteins, surfactants, and salts. The lateral flow strip may be a porous matrix composed of nitrocellulose, modified nitrocellulose, polyethersulfone, cellulose, glass fiber, polyvinylidene fluoride, or nylon. The lateral flow strip has at least one detection region composed of affinity pairs specific to the iSDA reaction products.

The practice of the methods described herein will employ, unless otherwise indicated, conventional techniques in organic chemistry, biochemistry, oligonucleotide synthesis and modification, bioconjugate chemistry, nucleic acid hybridization, molecular biology, microbiology, genetics, recombinant DNA, and related fields as are within the skill of the art. These techniques are fully explained in the literature. See, for example, Sambrook, Fritsch & Maniatis, MOLECULAR CLONING: A LABORATORY MANUAL, Second Edition, Cold Spring Harbor Laboratory Press (1989); Ausubel, et al., CURRENT PROTOCOLS IN MOLECULAR BIOLOGY, John Wiley & Sons (1987, 1988, 1989, 1990, 1991, 1992, 1993, 1994, 1995, 1996); Gait (ed.), OLIGONUCLEOTIDE SYNTHESIS: A PRACTICAL APPROACH, IRL Press (1984); and Eckstein (ed.), OLIGONUCLEOTIDES AND ANALOGUES: A PRACTICAL APPROACH, IRL Press (1991).

### III. Descriptions

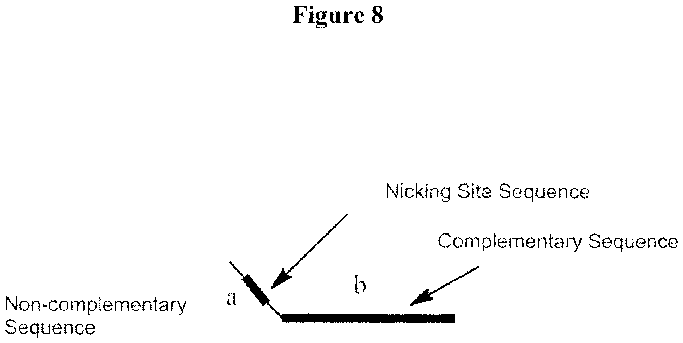

In one aspect, this disclosure provides an isothermal method for specifically detecting a nucleic acid sequence in a biological sample from an individual. The isothermal method can be carried out entirely at room temperature, or between about 40°C and about 65°C, or more preferably between about 45°C and about 55°C. The disclosure also provides oligonucleotide primers and probes comprising nucleotide sequences characteristic of a specific genomic nucleic acid sequences. The method includes performing of isothermal amplification without a denaturation step prior to amplification. The amplification step includes contacting the sample nucleic acid with pairs of primers to produce amplification product(s) if the specific genomic nucleic acid target is present. The primer "a-b" comprises a complementary sequence "b" and comprises a non-complementary nicking enzyme recognition sequence site "a" when hybridized to a complementary sequence (Figure 8). Primer a-b further comprises sequences selected by free energy minimization for specific hybridization and efficient elongation. The primers target a specific region of a specific target gene that allows amplification without thermal denaturation. Bumper primers hybridize upstream of the 5'-end of the flap primers to generate a target specific single stranded DNA newly synthesized amplicon by strand displacement (Nuovo GJ, .Diagn Mol Pathol. 2000 Dec;9(4): 195-202.). The oligonucleotide probes detect the amplified target directly or indirectly. The preferred oligonucleotide probe is a 5'-minor groove binder-fluorophore-oligonucleotide-quencher-3' conjugate that fluoresces on hybridization to its complementary amplified target.

In some embodiments the probe(s) is omitted. In some embodiments the amplified target is captured on a solid support, surface or membrane and detected by a labeled probe. In some embodiments the primer concentrations are present in different concentrations. In some embodiments an internal control is provided.

In a particular embodiment human, animal, and/or plant pathogen nucleic acids are amplified and detected.

In another embodiment the amplified target nucleic acid is RNA and the method further comprises a reverse transcriptase step.

In another aspect, the 5' non-complementary sequence comprises a sequence for a nicking site. Although any enzyme with a suitable nicking site can be used, preferred nicking enzyme recognition sequences are selected from N.Alw I, Nb.BbvCl, Nt.BbvCl, Nb.BsmI, Nt.BsmAI, Nt.BspQI, Nb.BsrDI, Nt.BstNBI, Nb.BstsCI, Nt.CviPII, Nb.Bpu10I, Nt.Bpu10I and Nt.Bst9I, Nb.Mva1269I and endo nuclease V.

In another embodiment, a complementary primer sequence comprises a sequence with an Endonuclease V ("Endo V") cleavage site requiring no heat or chemical denaturation, as more fully described in U.S. Patent No. 8,202,972 or U.S. Patent Application Publication No. 2011/0171649, which describes Endo V-based amplification primers. More specifically Endonuclease V is a repair enzyme recognizing DNA oligonucleotides containing deaminated modified bases such as inosine. Endo V cleaves the second or third phosphodiester bond 3' to the modified base, such as inosine. U.S. Patent No. 8,202,972 describes an Endonuclease V-based amplification method that extends a forward- and reverse-primer containing inosine adjacent to 3'-end terminal base. In the second round of amplification the Endo V cleaves the second or third phosphodiester bond 3' to the inosine in the same strand. The 3'-hydroxyl of the nick is extended by DNA polymerase in a template-directed manner. Employing a series of nested primer pairs complementary upstream of the 5'-end of the inosine containing primer pair, a series of extension products are generated. U.S. Patent No. 8,202,972 requires that "target dsDNA may be thermally denatured, chemically denatured, or both thermally and chemically denatured".

### EXAMPLES

The following examples are provided to illustrate, but not to limit, the subject matter described herein.

In these examples, iSDA was performed using final concentrations of 3.75 mM MgSO₄, 50 mM KH₂PO₄ pH 7.6, 250 nM forward primer, 1 µM reverse primer, 50 nM bumper oligonucleotides, 200 nM probe, 0.2 mM dNTPs, 40 µg/mL BSA, 10 ng genomic DNA, 4U N.BbvC1B and 3.6U Bst DNA polymerase in a total volume of 20 µL (mono-reagent). Twenty microliters of the mono-reagent was introduced in a 96 well PCR plate with 10 µL of sample nucleic acid. Sample nucleic acid was obtained by extraction with easyMag using NucliSENSE easyMAG extraction reagents (Biomerieux, l'Etoile, France). The plate was sealed with MicroAmp® Optical Adhesive Film (Applied Biosystems, Foster City, CA) and then centrifuged to collect the assay solution in the bottom of the plate well. The assay was then performed in an ABI 7500 DX Fast Block Real-time PCR machine at 48°C for 30 minutes.

### EXAMPLE 1.

This example demonstrates the efficient iSDA amplification without denaturation of the *ldh1* gene from easyMag extracted nucleic acid from cultured *S*. *aureus subsp. aureus COL* (gi|57650036 :262250-263203). The primer, bumper and probe sequences are shown in Table 1.

Table 1 below illustrates *ldh1* oligonucleotide sequences for iSDA amplification. Underlined sequences represents the nicking site for N.BbvC1B. The upper case sequence is *ldh1* specific, the 5'-end lower case sequence is non-complementary to the *ldh1* target, and the pDNA sequence is shown in brackets. Q14 is a hexaethylene glycol linker, MGB is a DPI₃ minor groove binder, FAM is fluorescein, and EDQ is the Eclipse® dark quencher (quenching range 390-625 nm, maximum absorption 522 nm, Epoch Biosciences, Inc., Bothell, WA).

**Table 1**

| Seq ID # | Description | Oligonucleotide sequence |
|---|---|---|
| 1 | Forward Primer | gcataatactaccagtctcctcagcAAGCTACGCATTTTCATTAG |
| 2 | Reverse Primer | tagaatagtcgcatacttcctcagcCATAACATCTCCTCGAACT |
| 3 | Probe | MGB-FAM-CTAATTCATCAACAATGC-EDQ |
| 4 | Forward Bumper | AGGTAATGGTGCAGTAGGT |
| 5 | Reverse Bumper | CCAGCTTTCACACGAAC |
| 6 | pDNA Capture Probe | [TTTTTTTTC]-(Q14)-CAGTGTCTAAATCAATGATG |
| 7 | Biotinilated Detection Probe | CTAATTCATCAACAATGC-biotin |

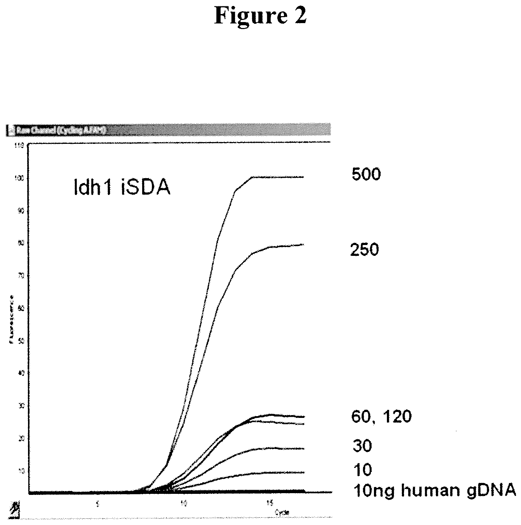

Real-time iSDA amplification with oligonucleotide 1 to 5 was performed as described above with target concentrations ranging from 10 to 500 copies per reaction. The results are shown in Figure 2.

### Lateral Flow:

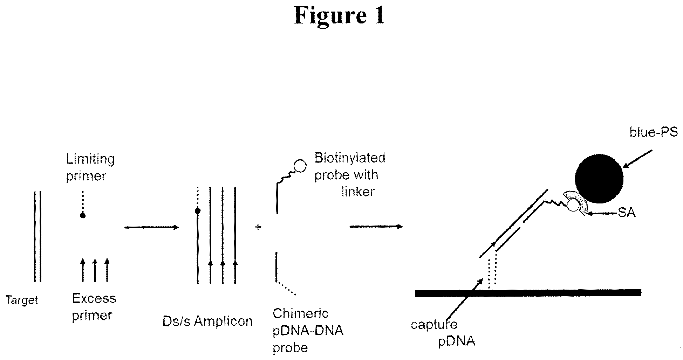

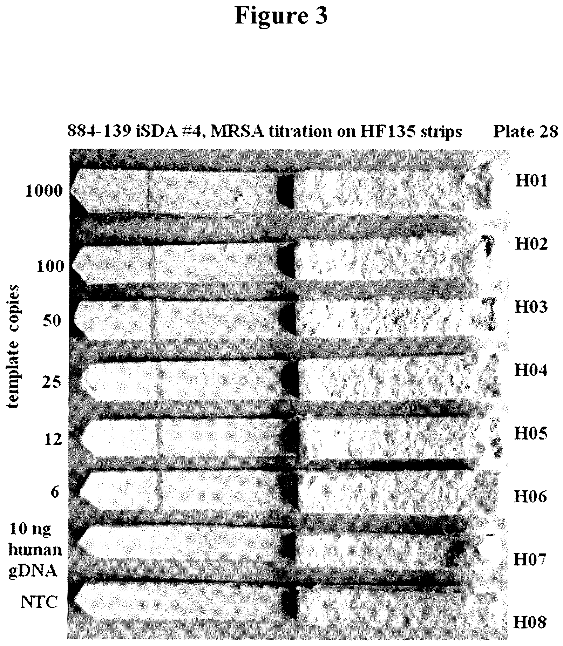

A similar iSDA amplification was performed except that probe 3 was replaced with probes 6 and 7 that allow capture and detection in a lateral flow format, as schematically depicted in Figure 1, with the results shown in Figure 3. Once the iSDA reaction was complete, 2 µL of the product was aliquoted into a well containing a streptavidin-coated label and a volume of buffer for running the lateral flow assay on HF135 nitrocellulose (Millipore), then the lateral flow strip was added to the well. In one example, 2 µL of the iSDA *ldh1* reaction mixture was diluted in 100 µL of lateral flow buffer with the formulation 15 mM HEPES (pH 8), 1% Triton X-100, 0.5% BSA, 400 mM NaCl, 0.05% NaN₃, and 100 ng/µL streptavidin-coated 300 nm diameter blue-dyed polystyrene nanoparticles (Seradyn). To the diluted product was then added a nitrocellulose strip, 4 x 25 mm, containing an immobilized pDNA oligo complementary to the pDNA capture probe 6. The pDNA was immobilized via a cross-linked polythymidine tail at a concentration of 120 pmol/cm and a line width of approximately 1 mm. Positive results were visualized easily by the naked eye (as seen in Figure 3).

### EXAMPLE 2.

This example illustrates the versatility of the design of primers from *mecA* gene sequences to allow iSDA amplification without denaturation. Nucleic acid was easyMag extracted from cultured *S. aureus subsp. aureus COL.* The primer, bumper and probe sequences of Design 1 and 2 are shown below in Table 2. The pDNA sequence is shown in brackets.

Table 2 below shows Designs 1 and 2 oligonucleotide sequences for *mecA* amplifications. Underlined sequences represent the nicking site for Nt.BbvC1B, the upper case sequence is *mecA* specific, the 5'-end lower case sequence is non-complementary to the *mecA* target, the pDNA sequence is shown in brackets, A* is Super A (US 7,045,610), and Q14 is a hexaethylene glycol linker.

**Table 2**

| **Seq ID #** | **Description** | **Oligonucleotide sequence** |
|---|---|---|
| *Design 1* | | |
| 8 | Forward Primer | gaaacaatgtacctgtcacctcagcGACCGAAACAATGTGGAAT |
| 9 | Reverse Primer | ttcaatagtcagttacttcctcagcGGAACGATGCCTAATCTCA |
| 10 | Probe | MGB-FAM-CCAATACAGGAACACAT-EDQ |
| 11 | Forward Bumper | GAAAATTTAAAATCAGAACGTGG |
| 12 | Reverse Bumper | GCTTTA*TAATCTTTTTTAGATAC |
| 13 | pDNA Capture Probe | [TTTTTTTTC]-(Q14-CAATGTGGA*ATTGG |
| 14 | Biotinilated Detection Probe | CCAATACAGGAACACAT-biotin |

| *Design 2* | | |
|---|---|---|
| 15 | Forward Primer | ccattatactacctgtctcctcagcGGCAAAGATATTCAACTAAC |
| 16 | Reverse Primer | tagaatagtcagttacttcctcagcGCCATAATCATTTTTCATGTTG |
| 17 | Probe | MGB-FAM-CTTTTGAACTTTAGCATC-EDQ |
| 18 | Forward Bumper | GATAATAGCAATACAATCGCACA |
| 19 | Reverse Bumper | GTGCTAATAATTCACCTGTTTGA |
| 20 | pDNA Capture Probe | [CAAGAATC]-(Q14)-CTTTAGCATCAATAGTTAG |
| 21 | Biotinilated Detection Probe | GTTA*TAAATA*CTCTTTTGA-biotin |

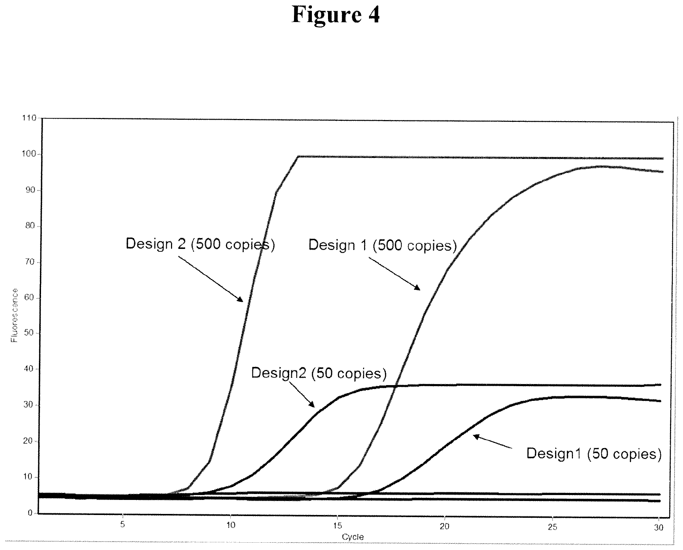

Using primers, probe and bumper oligonucleotides (Design 1, Seq. ID# 8-12 and Design 2, Seq. ID # 15-18) in the same way described in Example 2, efficient real-time iSDA was achieved as shown in Figure 4.

### EXAMPLE 3.

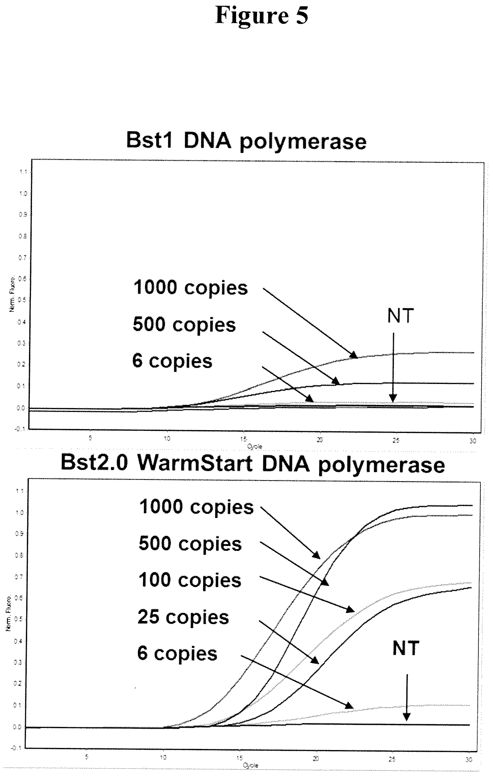

This example demonstrates the use of different polymerases in the real-time iSDA amplification. iSDA amplification was performed as described above using either *Bst* DNA Polymerase (portion of *Bacillus stearothermophilus* DNA Polymerase, New England BioLabs Inc., Ipswich, MA) or *Bst2.0* WarmStart (an *in silico* designed homologue of *Bacillus stearothermophilus* DNA Polymerase I, New England BioLabs Inc.). The latter enzyme amplified *mecA* target and is active above 45°C. The results are shown in Figure 5, indicating better performance with the *Bst2.0* WarmStart enzyme.

### EXAMPLE 4.

This example demonstrates that although the Nt.Alw1 nicking enzyme successfully cut a PCR amplicon into which the NtAlw1 nicking site was designed, it did not cut extracted genomic DNA even though the *ldh1* gene contains a natural nicking site for NtAlw1.

The sequences below in Table 3 were used to incorporate a nicking site into a PCR amplicon. The ldh1 specific sequences were designed with traditional PCR design software.

In Table 3 below, Design 3 and 4 oligonucleotide sequences for *ldh1* amplifications were generated with the Eclipse Design Software 2.3. Underlined sequences represent the nicking site for NtAlwl, the upper case sequence is *ldh1* specific, and the 5'-end lower case sequence is non-complementary to the *ldh1* target.

**Table 3**

| **Seq ID #** | Description | Oligonucleotide sequence |
|---|---|---|
| *Design 3* | | |
| 22 | Limiting primer-L 1 | aataaatcataaggatcAACGTGTTATAGGTTCTGGTACA |
| 23 | Excess primer -E1 | aataaatcataaggatcTGAGCATCGACGCTACGTG |
| 24 | Forward Bumper1 | ATGGAAATTCTCTGGT |
| 25 | Reverse Bumper1 | TGTCACCATGTTCAC |

| *Design 4* | | |
|---|---|---|
| 26 | Limiting primer-L2 | aataaatcataaggatcTGGTGAACATGGTGACACTGAAT |
| 27 | Excess Primer E2 | aataaatcataaggatcGCCCTCAGGACGTTGTTCAAG |
| 28 | Forward Bumper2 | AGCGTCGATGCTCA |
| 29 | Reverse Bumper2 | AATTTGTTCAATTTGCG |

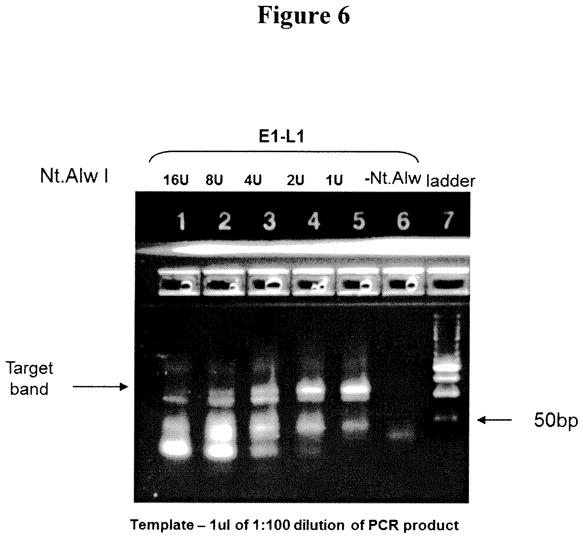

Primers of Design 3 and Design 4 were used to generate PCR amplicons which contain a nicking site for NtAlw1, yielding a convenient target containing a nicking site for NtAlw1. iSDA with the PCR-generated amplicon was analyzed on an agarose gel and the results are shown in Figure 6.

### EXAMPLE 5.

This example illustrates the iSDA bi-plexing of *ldh1* and an internal control ("IC"). The IC template contains nonsense, non-specific target DNA fragment in a plasmid vector. Preferably, the control nucleic acid comprises the sequence shown in Table 4 below.

In Table 4 below, oligonucleotide sequences for the amplification of the IC were generated as described above for iSDA amplification. Underlined sequences represent the nicking site for Nt.BbvC1B, the upper case sequence is IC- specific, and the 5'-end lower case sequence is non-complementary to the IC target. The same *ldh1* primers, bumper, capture and detection oligonucleotides (Seq. ID# 1, 2 4-7, Table 1) were used for the bi-plexing of the *Idh1* with the IC . The IC primers, bumpers, capture and detection probes sequences are shown in Table 4.

**Table 4**

| **Seq ID #** | Description | Oligonucleotide sequence |
|---|---|---|
| | | |
| 30 | Limiting primer-L 1 | ccaatatagtaacagtctcctcagcATTCGCCCTTCTGCACG |
| 31 | Excess primer-E1 | ttcaaaagacccatacttcctcagCCTTCTCATTTTTTCTACCG |
| 32 | Forward Bumper1 | TCGGATCCACTAGTAAC |
| 33 | Reverse Bumper1 | GTGATGGATATCTGCAGAAT |
| 34 | Chimeric pDNA/DNA | [ACATCACA]-Q14-GATCTTGTACCAATGC |
| 35 | Biotinilated probe | CGTGGTCCGTAAAG -biotin TEG |
| 36 | IC2 | TTTCACACAGGAAACAGCTATGACCATGATTACGCCAAGCTATTTAG GTGACACTATAGAATACTCAAGCTATGCATCAAGCTTGGTACCGAGC TCGGATCCACTAGTAACGGCCGCCAGTGTGCTGGAATTCGCCCTTCT GCACGGACCAGTTACTTTACGGACCACGTACCGCATTGGTACAAGATC TCCGGTAGAAAAAATGAGAAGGGCGAATTCTGCAGATATCCATCACA |
| | | CTGG |

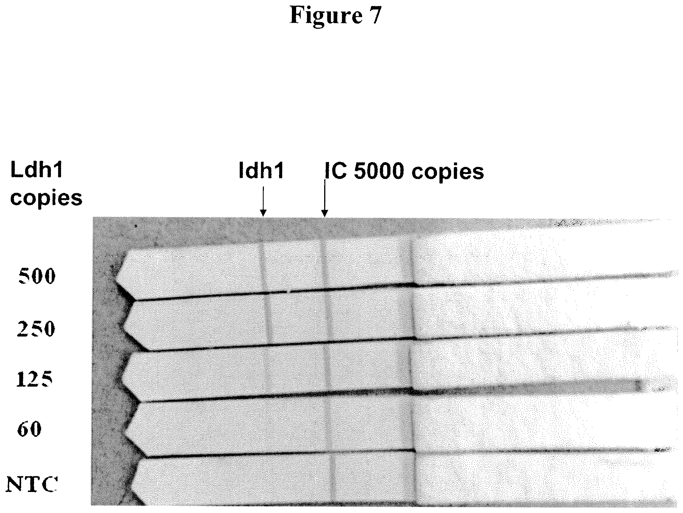

iSDA amplification was performed as described above, except that the concentration for both *ldh1* and the IC primers were 250nM for the limiting primer and 500nM for excess primer, forward and reverse bumper primers were at 50nM, the chimeric pDNA-DNA probe and biotinylated probe at 200nM each. Each target dilution contained 5000 IC2 copies. The amplification reaction was incubated at 48°C for 30 minutes then it was analyzed by lateral flow analysis as described above. The lateral flow analysis is shown in Figure 7 indicating for this particular assay a lower detection limit of 60 copies.

### EXAMPLE 6

This example illustrates the probe specific iSDA detection and differentiation of *S.aureus* (BAA-1556, ATCC) and *S.epidermidis* (12228, ATCC).

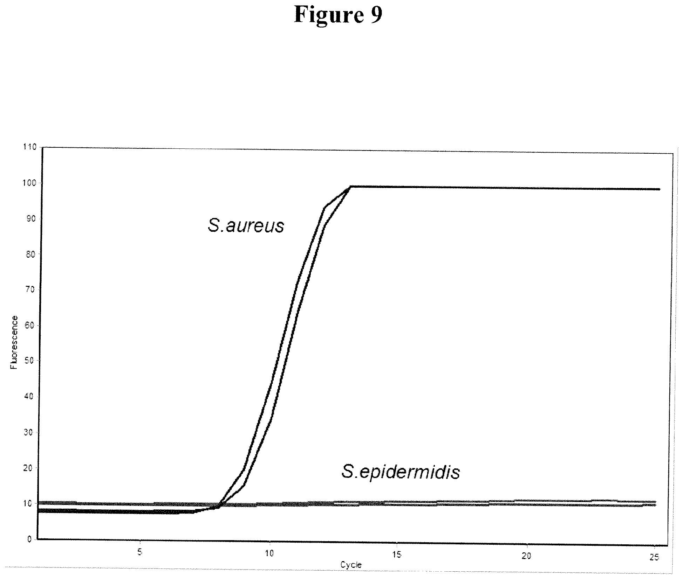

Cultures of *S.aureus and S. epidermidis* (5x10⁸ cfu/mL) were sonicated for 10 min in the waterbath sonicator (Branson 5510, Bransonic) The crude lysates were assayed for the *ldh1* gene according to the method described in Example 1 at a concentration of 5x10⁴ cfu/reaction. Efficient specific detection of the *ldh1* gene in *S. aureus* only is shown in Figure 9.

### EXAMPLE 7

This example illustrates the iSDA amplification of nucleic acid from the same sample extracted with different methods.

A *S. aureus* sample was extracted using the following extraction methods:
a) Extraction with chaotropic salts (8M guanidinium HCl or 4M guanidinium thiocyanate), with and without the silica spin column.
   Bacterial cells (5x10⁸ cfu) were extracted according to the procedure described in Molecular Cloning: a laboratory manual. (pages 7-19, 7-24). DNA from each extraction was resuspended in 200µL of the TE buffer and divided into two 100µL aliquots. One aliquot was set aside for PCR and iSDA analysis, and another one was further purified on QIAmp DNA Mini Kit (Qiagen) spin columns according to the product manual. DNA was eluted in 100 µL of the elution buffer.
b) Phenol/chloroform extraction followed by ethanol precipitation. (Molecular Cloning: a laboratory manual, App.E3-E4).
c) Sonication for 10 min in the waterbath sonicator(Branson 5510, Bransonic).
d) 10% final concentration of Triton X100 incubation at room temperature followed by ethanol precipitation.

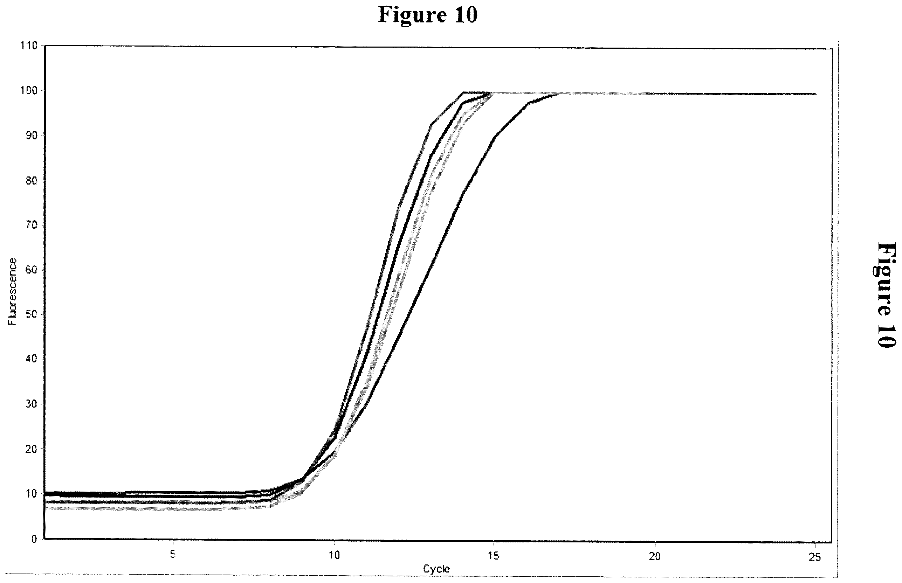

The concentrations of different non-denatured DNA nucleic acid fractions were normalized at 500 copies/reaction by real-time *ldh1* PCR assay (described in U.S. Patent Application Serial No. 13/479,557). As shown in Figure 10, all five extractions gave essentially the same signal result at around cycle 9 (9 min). The NTC showed no amplification and is not shown.

### EXAMPLE 8

This example illustrates the iSDA amplification of the *ldh1* gene with primers and probes designed with the current disclosure in comparison with traditional designed primers and probes shown in Table 5

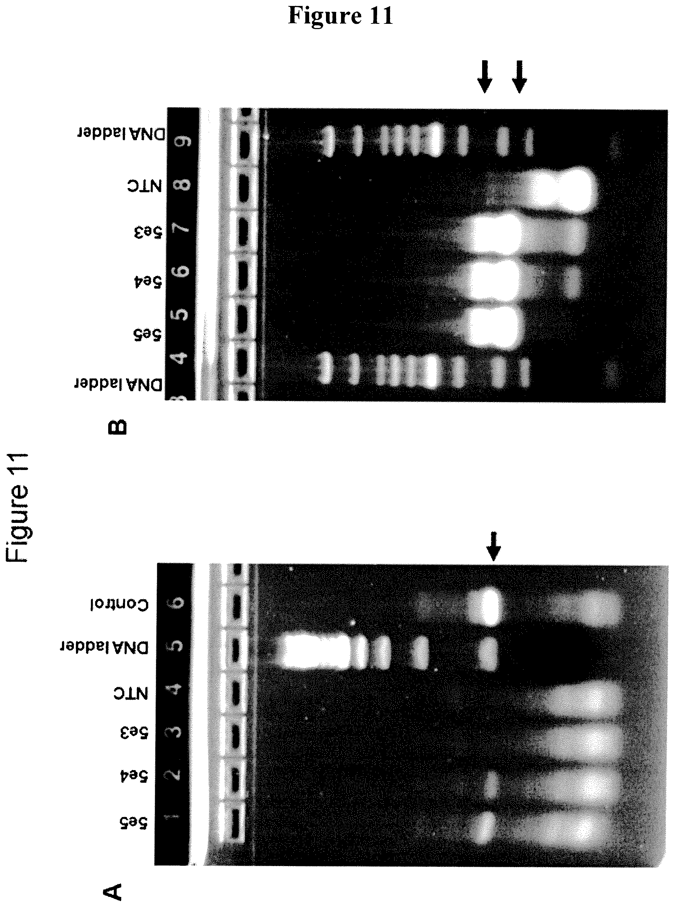

Using the method described in Example 1, the primers and bumper primers for the *ldh1* gene described in Tables 1 and 5 were tested in which both sets of primers had target concentrations ranging from 5 x10³ to 5 x10⁵ target copies/reaction. The amplification reactions were analyzed by agarose gel electrophoresis as shown in Figure 11A and B. The arrows in Figure 11A and B refer to the amplicon products of amplification. As shown the amplification with the primers of the current disclosure showed substantial amplification at all three concentrations, while the conventional designed primers showed poor amplification Figure 11A.

**Table 5**

| **Seq ID #** | Description | Oligonucleotide sequence |
|---|---|---|
| | | |
| 37 | Limiting primer-L1 | gcattatagtacctgtctcctcagcTGGTGAACATGGTGACACTGAAT |
| 38 | Excess primer-E1 | ttgaatagtcggttacttcctcagcGCCCTCAGGACGTTGTTCAAG |
| 39 | Forward Bumper1 | AGCGTCGATGCTCA |
| 40 | Reverse Bumper1 | AATTTGTTCAATTTGCG |

### EXAMPLE 9

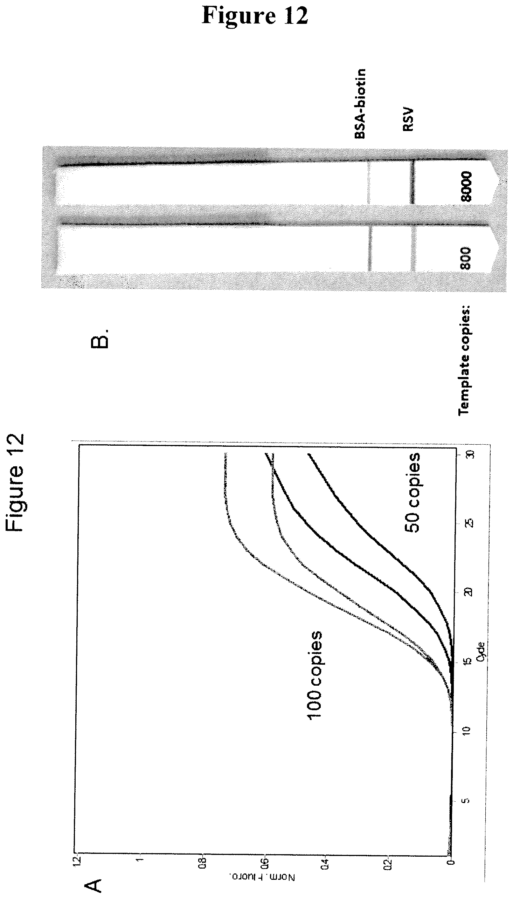

This example illustrates the one step RT-iSDA amplification of RSV nucleic acid. RT-iSDA uses the same final concentrations as disclosed for iSDA in [0049], except that 8U WarmStart Bst Polymerase was substituted for Bst Polymerase, 8U Nt.BbvC1 nicking enzyme was used per 10 µL reaction. In addition the reaction mixture contains 10U RNA inhibitor (Life Technologies), 0.5 µL Omniscript Reverse Transcriptase (Qiagne), template RNA and 1 µg BSA per 10 µL/reaction. Reaction mixture was followed in real-time for 25 minutes at 49°C as illustrated in Figure 12a) and lateral flow detection in Figure 12b). Primers, bumper primers and probes are shown in Table 6 below. T*= Super T and other abbreviations have been described above. The lateral flow membrane has a test line of pDNA (immobilized by cross-linked polythymidine tail) and a BSA-biotin line as flow control.

**Table 6**

| **Seq ID #** | Description | Oligonucleotide sequence |
|---|---|---|
| | | |
| 41 | Limiting primer-L1 | gcattatagtacctgtctcctcagcGAATTCCCTGCATCAATAC |
| 42 | Excess primer-E1 | gcattatggtacctctctcctcagcTA*TGTCA*ATATCT*T*CATC |
| 43 | Forward Bumper1 | AACTAAGGCCAAAGCTTATAC |
| 44 | Reverse Bumper1 | CAGTCAGTAGTAGACCATG |
| 45 | Chimeric pDNA/DNA | [TTTTTTTTC]-(Q14)-CTACAAATTATCACTTTGA |
| 46 | Biotinilated probe | TA*ATCGCATATTAACAG-biotin TEG |
| 47 | FAM probe | MGB-FAM-TAATCGCATAT*T*AACAG-EDQ |

### EXAMPLE 10

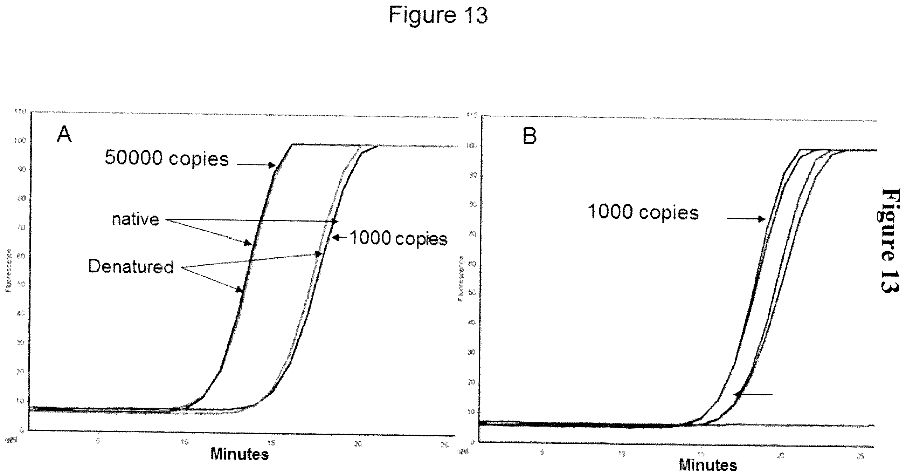

This example illustrates the iSDA amplification of native and denatured *P.falciparum genomic* DNA. Primers and probes were designed using mitochondrial DNA (Polley et.al,. J. Clin. Microbiol, 48:2866-2871 (2010)) as a target and is shown in Table 7 below. Extraction from *Plasmodium falciparum,* strain NF54 and iSDA amplification were performed as described above. Figure 13A shows identical real-time iSDA amplification for native and denature DNA at 95°C for 5 minutes. Figure 13B shows the amplification of native DNA at 100 and 1000 copies.

**Table 7**

| **Seq ID #** | Description | Oligonucleotide sequence |
|---|---|---|
| | | |
| 48 | Limiting primer-L1 | gaatagacccatacatcctcagcGACTTGAGTAATGATAAATTGATAG |
| 49 | Excess primer -E1 | gaatagacccatacatcctcagcGACTTGAGTAATGATAAATTGATAG |
| 50 | Forward Bumper1 | CCA*CTTGCTTATAACTGTATG |
| 51 | Reverse Bumper1 | GTTTCCA*TAGAAACCTTCAT |
| 52 | FAM probe | MGB-FAM-ATTGATTCCGTTTTGAC-EDQ |

### REFERENCES CITED

U.S. and Foreign Patent Documents
US 5,824,796
US 5,912,340
US 5,455,166
US 7,282,328
US 4,943,522
US 7488578
US Application Publication No. 2012-0015358 A
US 5,624,825
US RE39885
US 5,455,166
US 5,422,252
US 5,624,825
US 5,712,124
US 5,270,184
US 5,470,723
US 5,561,944
US 5,736,365
US 6,127,121
US 6,660,845
US 6683173
US 7045610
US 7751982
US 7,799,554
US 8,202,972
U.S. Patent Application Publication No. 2010/057862
U.S. Patent Application Publication No. 2011/0171649
U.S. Patent Application Publication No. 2012/0244535
PCT Publication WO 01/38584
PCT Publication WO 01/64958

### Non-Patent References

Afonina et al., Single Nucleotide Polymorphism Detection with fluorescent MGB Eclipse Systems in A-Z of Quantitative PCR, Ed. S. A. Bustin, International University Line, La Jolla, CA, pages 718-731 and XII-XIII, 2004)
Besnier and Kong, EMBO Reports, 21: 782-786 (2001)
Ehses et al., J. Biochem. Biophys. Methods. 63:170-86 (2005).
Eschenmoser et al, Helvetica Chimica Acta, 76: 2161-2183 (1993)
Fran-Kamentskii. Artificial DNA; PNA & XNA, 2:1, 1-3 (2011)
Molecular Cloning: a laboratory manual
Niemz et al., Trends in Biotechnol., 29:240-250 (2011))
Nuovo, Diagn Mol Pathol. 9(4):195-202 (2000)
M Panaccio and A Lew. PCR based diagnosis in the presence of 8% (v/v) blood. Nucleic Acids Res., 19: 1151 (1991)
Polley et al, J. Clin. Microbiol, 48:2866-2871 (2010)
Ramirez et al, Nucl. Acids Res., 40:5560-8 (2012)
Roberts et al., Nucl. Acids Res., 31: 418-420 (2003)
Walker et al., NAR 20: 1691- 1695 (1992)
Walker, PCR Methods and Applications, 3: 1-6 (1993)
Walker et al., NAR 22: 2670 (1994)
Walker et al., Clin. Chem., 42: 9-13 (1996)
Walker et al., Clin. Chem., 42: 1604-8 (1996)
Walker et al., NAR 24:349 (1996)
Wang et al., Clin. Chem., 49: 1599 (2003)
White, Handler and Smith, Principles of Biochemistry 5th Edition, McGraw-Hill Kogakush, Ltd, pages 192-197, 1993
Xu et al, PNAS 98: 12990-12995 (2001)
Zheleznaya et al., Biochemistry (Mosc). 74:1457-66 (2009)

## Claims

1. An isothermal strand displacement amplification method, the method comprising:
(a) contacting a double stranded genomic nucleic acid having a target nucleic acid sequence with an amplification reaction mixture comprising:
a forward primer and a reverse primer,
wherein the forward primer has the formula:
A-B,
wherein B comprises a portion of the forward primer that is complementary to the target nucleic acid sequence, and wherein A comprises a portion of the forward primer that is non-complementary to the target nucleic acid sequence and comprises a forward nicking enzyme recognition sequence,
wherein the reverse primer has the formula:
A'-B',
wherein B' comprises a portion of the reverse primer that is complementary to the target nucleic acid sequence, and wherein A' comprises a portion of the reverse primer that is non-complementary to the target nucleic acid sequence and comprises a reverse nicking enzyme recognition sequence, and
wherein the forward primer and the reverse primer comprise sequences optimized by software for specific hybridization and efficient elongation,
a polymerase enzyme having strand displacement activity, and
a nicking enzyme specific for the nicking enzyme recognition sequence;
(b) incubating the amplification reaction mixture and the double stranded genomic nucleic acid under amplification conditions suitable for amplification of the target nucleic acid to produce an amplified target nucleic acid,
wherein isothermal amplification of the target nucleic acid occurs at a temperature between about 45°C and about 55°C and the double stranded genomic nucleic acid is not denatured prior to amplification; and
(c) detecting the amplified target nucleic acid.

2. The method of claim 1 wherein the amplification reaction mixture further comprises one or more bumper oligonucleotides or an internal control.

3. The method of claim 1 wherein the step of detecting the amplified target nucleic acid comprises using fluorescence resonance energy (FRET), radiolabels, lateral flow, or enzyme labels.

4. The method of claim 1 wherein the step of detecting the amplified target nucleic acid comprises hybridizing an oligonucleotide probe to at least a portion of the amplified target nucleic acid.

5. The method of claim 4 wherein the oligonucleotide probe comprises a minor groove binder (MGB), a fluorophore, and a quencher.

6. The method of claim 4 wherein the oligonucleotide probe fluoresces when hybridization to the amplified target nucleic acid occurs.

7. The method of claim 4 wherein the oligonucleotide probe is cleaved to produce a fluorescent signal.

8. The method of claim 1 wherein at least one of the forward primer and reverse primer comprises a fluorescent label.

9. The method of claim 1 wherein the step of detecting the amplified target nucleic acid comprises attaching the amplified target nucleic acid to a solid support and detecting the amplified target nucleic acid with an oligonucleotide probe having a fluorescent label.

10. The method of claim 1 wherein the software comprises the Vienna Folding Package.

11. The method of claim 1 wherein the software comprises software for adjusting the Tₘ of the forward primer and the reverse primer by calculating duplex stabilities using an algorithm applying a nearest-neighbor model for duplex formation thermodynamics for each neighboring base pair.

12. The method of claim 1 wherein the forward primer and the reverse primer are present in different concentrations in the amplification reaction mixture.

13. The method of claim 1 wherein at least one of the forward primer and reverse primer is substituted with at least one modified base.

14. The method of claim 1 wherein at least one of the forward nicking enzyme recognition sequence and the reverse nicking enzyme recognition sequence comprises a cleavage site for Endonuclease V.

15. A method of claim 1 wherein A' comprises a sequence for a cleavage site.

## Patentansprüche

1. Verfahren zur isothermen Strangverdrängungsamplifikation, wobei das Verfahren Folgendes beinhaltet:
(a) Inkontaktbringen einer doppelsträngigen genomischen Nucleinsäure, die eine Zielnucleinsäuresequenz hat, mit einem Amplifikationsreaktionsgemisch, das Folgendes umfasst:
einen Vorwärtsprimer und einen Rückwärtsprimer,
wobei der Vorwärtsprimer die folgende Formel hat:
A-B,
wobei B einen Teil des Vorwärtsprimers umfasst, der komplementär zur Zielnucleinsäuresequenz ist, und wobei A einen Teil des Vorwärtsprimers umfasst, der nichtkomplementär zur Zielnucleinsäuresequenz ist und eine Vorwärts-Nicking-Enzym-Erkennungssequenz umfasst,
wobei der Rückwärtsprimer die folgende Formel hat:
A'-B',
wobei B' einen Teil des Rückwärtsprimers umfasst, der komplementär zur Zielnucleinsäuresequenz ist, und wobei A' einen Teil des Rückwärtsprimers umfasst, der nichtkomplementär zur Zielnucleinsäuresequenz ist und eine Rückwärts-Nicking-Enzym-Erkennungssequenz umfasst, und
wobei der Vorwärtsprimer und der Rückwärtsprimer Sequenzen umfassen, die durch eine Software für eine spezifische Hybridisierung und effiziente Verlängerung optimiert sind,
ein Polymeraseenzym mit Strangverdrängungsaktivität und
ein Nicking-Enzym, das für die Nicking-Enzym-Erkennungssequenz spezifisch ist;
(b) Inkubieren des Amplifikationsreaktionsgemischs und der doppelsträngigen genomischen Nucleinsäure unter Amplifikationsbedingungen, die für eine Amplifikation der Zielnucleinsäure geeignet sind, um eine amplifizierte Zielnucleinsäure zu produzieren,
wobei die isotherme Amplifikation der Zielnucleinsäure bei einer Temperatur zwischen etwa 45°C und etwa 55°C stattfindet und die doppelsträngige genomische Nucleinsäure vor der Amplifikation nicht denaturiert wird; und
(c) Detektieren der amplifizierten Zielnucleinsäure.

2. Verfahren nach Anspruch 1, wobei das Amplifikationsreaktionsgemisch ferner ein oder mehrere Bumper-Oligonucleotide oder eine interne Kontrolle umfasst.

3. Verfahren nach Anspruch 1, wobei der Schritt des Detektierens der amplifizierten Zielnucleinsäure die Verwendung von Fluoreszenzresonanzenergie (FRET), Radiomarkierungen, Lateralfluss oder Enzymmarkierungen beinhaltet.

4. Verfahren nach Anspruch 1, wobei der Schritt des Detektierens der amplifizierten Zielnucleinsäure das Hybridisieren einer Oligonucleotidsonde mit wenigstens einem Teil der amplifizierten Zielnucleinsäure beinhaltet.

5. Verfahren nach Anspruch 4, wobei die Oligonucleotidsonde einen Kleine-Furche-Binder (MGB), ein Fluorophor und einen Quencher umfasst.

6. Verfahren nach Anspruch 4, wobei die Oligonucleotidsonde fluoresziert, wenn eine Hybridisierung mit der amplifizierten Zielnucleinsäure stattfindet.

7. Verfahren nach Anspruch 4, wobei die Oligonucleotidsonde gespalten wird, um ein Fluoreszenzsignal zu erzeugen.

8. Verfahren nach Anspruch 1, wobei der Vorwärtsprimer und/oder der Rückwärtsprimer eine fluoreszierende Markierung umfasst/umfassen.

9. Verfahren nach Anspruch 1, wobei der Schritt des Detektierens der amplifizierten Zielnucleinsäure das Anlagern der amplifizierten Zielnucleinsäure an einen festen Träger und das Detektieren der amplifizierten Zielnucleinsäure mit einer Oligonucleotidsonde mit einer fluoreszierenden Markierung beinhaltet.

10. Verfahren nach Anspruch 1, wobei die Software das Vienna Folding Package umfasst.

11. Verfahren nach Anspruch 1, wobei die Software Software zum Einstellen des Tₘ des Vorwärtsprimers und des Rückwärtsprimers durch Berechnen von Duplex-Stabilitäten unter Verwendung eines Algorithmus umfasst, der ein Nächste-Nachbarn-Modell für die Duplexbildungs-Thermodynamik für jedes benachbarte Basispaar anwendet.

12. Verfahren nach Anspruch 1, wobei der Vorwärtsprimer und der Rückwärtsprimer im Amplifikationsreaktionsgemisch in unterschiedlichen Konzentrationen vorliegen.

13. Verfahren nach Anspruch 1, wobei der Vorwärtsprimer und/oder der Rückwärtsprimer durch wenigstens eine modifizierte Base substituiert wird.

14. Verfahren nach Anspruch 1, wobei die Vorwärts-Nicking-Enzym-Erkennungssequenz und/oder die Rückwärts-Nicking-Enzym-Erkennungssequenz eine Spaltstelle für Endonuclease V umfasst.

15. Verfahren nach Anspruch 1, wobei A' eine Sequenz für eine Spaltstelle umfasst.

## Revendications

1. Procédé d'amplification isotherme par déplacement de brin, le procédé consistant à :
(a) mettre en contact un acide nucléique génomique bicaténaire ayant une séquence d'acides nucléiques cible avec un mélange de réaction d'amplification comprenant :
une amorce sens et une amorce antisens,
l'amorce sens étant de formule :
A-B,
B comprenant une partie de l'amorce sens qui est complémentaire de la séquence d'acides nucléiques cible, et A comprenant une partie de l'amorce sens qui est non complémentaire de la séquence d'acides nucléiques cible et comprenant une séquence sens de reconnaissance d'enzyme de coupure,
l'amorce antisens étant de formule :
A'-B',
B' comprenant une partie de l'amorce antisens qui est complémentaire de la séquence d'acides nucléiques cible, et A' comprenant une partie de l'amorce antisens qui est non complémentaire de la séquence d'acides nucléiques cible et comprenant une séquence antisens de reconnaissance d'enzyme de coupure, et
l'amorce sens et l'amorce antisens comprend des séquences optimisées par logiciel pour une hybridation spécifique et un allongement efficace,
une enzyme polymérase ayant une activité de déplacement de brin, et
une enzyme de coupure spécifique de la séquence de reconnaissance d'enzyme de coupure ;
(b) incuber le mélange de réaction d'amplification et l'acide nucléique génomique bicaténaire dans des conditions d'amplification convenant à une amplification de l'acide nucléique cible pour produire un acide nucléique cible amplifié,
une amplification isotherme de l'acide nucléique cible se produisant à une température comprise entre environ 45 °C et environ 55 °C et l'acide nucléique génomique bicaténaire n'étant pas dénaturé avant l'amplification ; et
(c) détecter l'acide nucléique cible amplifié.

2. Procédé selon la revendication 1, dans lequel le mélange de réaction d'amplification comprend un ou plusieurs oligonucléotidiques butoirs ou un témoin interne.

3. Procédé selon la revendication 1, dans lequel l'étape de détection de l'acide nucléique cible amplifié consiste à utiliser une énergie par résonance de fluorescence (FRET), des marqueurs radioactifs, un écoulement latéral ou des enzymes marqueurs.

4. Procédé selon la revendication 1, dans lequel l'étape de détection de l'acide nucléique cible amplifié consiste à hybrider une sonde oligonucléotidique avec au moins une partie de l'acide nucléique cible amplifié.

5. Procédé selon la revendication 4, dans lequel la sonde oligonucléotidique comprend un lieur du sillon mineur (MGB), un fluorophore et un extincteur.

6. Procédé selon la revendication 4, dans lequel la sonde oligonucléotidique émet une lumière fluorescente quand l'hybridation avec l'acide nucléique cible amplifié se produit.

7. Procédé selon la revendication 4, dans lequel la sonde oligonucléotidique est clivée pour produire un signal fluorescent.

8. Procédé selon la revendication 1, dans lequel l'amorce sens et/ou l'amorce antisens comprennent un marqueur fluorescent.

9. Procédé selon la revendication 1, dans lequel l'étape de détection de l'acide nucléique cible amplifié consiste à attacher l'acide nucléique cible amplifié à un support solide et détecter l'acide nucléique cible amplifié avec une sonde oligonucléotidique ayant un marqueur fluorescent.

10. Procédé selon la revendication 1, dans lequel le logiciel comprend le package de repliement Vienna.

11. Procédé selon la revendication 1, dans lequel le logiciel comprend un logiciel pour régler la Tm de l'amorce sens et de l'amorce antisens en calculant des stabilités de duplex au moyen d'un algorithme appliquant un modèle du plus proche voisin pour la thermodynamique de formation de duplex pour chaque paire de bases voisines.

12. Procédé selon la revendication 1, dans lequel l'amorce sens et l'amorce antisens sont présentes en différentes concentrations dans le mélange de réaction d'amplification.

13. Procédé selon la revendication 1, dans lequel l'amorce sens et/ou l'amorce antisens sont remplacées par au moins une base modifiée.

14. Procédé selon la revendication 1, dans lequel la séquence sens de reconnaissance d'enzyme de coupure et/ou la séquence antisens de reconnaissance d'enzyme de coupure comprennent un site de clivage pour l'endonucléase V.

15. Procédé selon la revendication 1, dans lequel A' comprend une séquence pour un site de clivage.
